# EUROPÄISCHE PATENTSCHRIFT

(11) **EP 2 948 534 B1**
(45) Veröffentlichungstag und Bekanntmachung des Hinweises auf die Patenterteilung: **15.03.2017**
(21) Anmeldenummer: 14701722.2
(22) Anmeldetag: 24.01.2014
(51) Int. Cl.: C11B 9/00, A61Q 13/00, C07C 29/14, C11D 3/50

(54) **VERWENDUNG VON 4,8-DIMETHYL-3,7-NONADIEN-2-OL ALS RIECHSTOFF**
USE OF 4,8-DIMETHYL-3,7-NONADIEN-2-OL AS A PERFUME
UTILISATION DE 4,8-DIMÉTHYL-3,7-NONADIÈNE-2-OL EN TANT QUE PARFUM

(30) Priorität: 25.01.2013 EP 13152724
(43) Veröffentlichungstag der Anmeldung: 02.12.2015
(73) Patentinhaber: BASF SE, 67056 Ludwigshafen am Rhein (DE)
(72) Erfinder: RÜDENAUER, Stefan, 69469 Weinheim (DE); PELZER, Ralf, 37699 Fürstenberg (DE); KRAUSE, Wolfgang, 68782 Brühl-Rohrhof (DE); BOCRIS, Francis, 06000 Nizza (FR)
(86) Internationale Anmeldenummer: PCT/EP2014/051368
(87) Internationale Veröffentlichungsnummer: WO 2014/114733

(56) Entgegenhaltungen:
- EP-A1- 0 743 297
- JP-A- 2004 091 742
- US-A1- 2001 005 711

## Beschreibung

Die vorliegende Erfindung betrifft die Verwendung von (E)- oder (Z)-angereichertem 4,8-Dimethyl-3,7-nonadien-2-ol als Riechstoff, sowie Riechstoffkompositionen und parfümierte Artikel, die dieses umfassen, Verfahren zum Vermitteln, Modifizieren und/oder Verstärken einer rosenartigen Geruchsnote, sowie Verfahren zur Herstellung von (E)- oder (Z)-angereichertem 4,8-Dimethyl-3,7-nonadien-2-ol.

Trotz einer Vielzahl bereits vorhandener Riechstoffe besteht in der Parfümindustrie ständiger Bedarf an neuen Riechstoffen, die über ihre primären, nämlich geruchlichen (olfaktorischen), Eigenschaften hinaus zusätzliche positive Sekundäreigenschaften besitzen, wie z.B. eine effiziente Herstellungsweise, eine höhere Stabilität unter bestimmten Anwendungsbedingungen, eine höhere Ausgiebigkeit oder ein besseres Haftungsvermögen, oder aber durch Synergieeffekte mit anderen Riechstoffen zu besseren sensorischen Profilen führen.

Es besteht in der Parfümindustrie auch grundsätzlich ein Bedarf an weiteren Riechstoffen, die sich zur Herstellung von Riechstoffkompositionen und/oder parfümierten Artikeln eignen. Insbesondere besteht ein Bedarf an Riechstoffen, die durch die oben erwähnten technischen Eigenschaften zu einem erhöhten Nutzen in Riechstoffkompositionen führen. So können z.B. durch den Einsatz von Riechstoffen mit einer effizienten Herstellungsweise, einer höheren Stabilität und einem besseren sensorischen Profil die Einsatzmengen und die Anzahl von Riechstoffen in entsprechenden Formulierungen optimiert und/oder minimiert werden, was zu einer nachhaltigen Ressourcenschonung beim Parfümieren von Konsum- und Verbrauchsgütern führt.

Insbesondere besteht ein Bedarf an Riechstoffen und Riechstoffkompositionen mit rosigen Noten. Hierunter ist im Rahmen der vorliegenden Erfindung ein Geruch zu verstehen, der dem Geruch des natürlich vorkommenden Rosenöls bzw. wie dem seiner Bestandteile ähnlich ist.

Citral (3,7-Dimethyl-octa-2,6-dienal) wird in großem Umfang als Riechstoff verwendet. Citral verleiht ätherischen Ölen wie z.B. dem Zitronengrasöl, dem May-Chang Öl sowie den Schalen von Citrusfrüchten einen Zitronenduft. Es kann sowohl aus natürlichen Quellen isoliert oder synthetisch hergestellt werden, wobei es üblicherweise in Form eines Gemisches aus (E)-Isomer (Geranial) und (Z)-Isomer (Neral) anfällt.

Hydrierprodukte von Citral wie z.B. die ungesättigten Alkohole Geraniol ((E)-3,7-Dimethyl-2,6-octadien-1-ol) und Nerol ((Z)-3,7-Dimethyl-2,6-octadien-1-ol), werden ebenfalls als Duft und Aromastoffe eingesetzt. Die Isomere weisen einen rosenartigen und citrusartigen Duft auf, wobei das (E)-Isomer Geraniol als Hauptbestandteil in Geranium und Rosenöl enthalten ist, während das (Z)-Isomer Nerol als ein Hauptbestandteil im Bergamotteöl enthalten ist.
Die US 2001/0005711 A1 beschreibt Riechstoffmischungen, die 4,8-Dimethyl-3,7-nonadien-2-on enthalten. Geringe Konzentrationen dieses Moleküls in Riechstoffkompositionen führen zu frischen, abgerundeten Geruchsnoten. Die beschriebenen Riechstoffkompositionen können weitere Duftstoffe enthalten, wobei einige von vielen Zusatzstoffen z.B. 4,8-Dimethyl-3,7-nonadien-2-ol, Geraniol, Tetrahydrogeraniol, Nerol, Geranial und Neral sein können.
Die EP 1417896 A1 beschreibt ein Verfahren zur Anreicherung, Verstärkung, Modifizierung oder Verleihung eines Beeren- oder Citrusaromas oder Geschmacks in einem Nahrungsmittel oder einem wasserhaltigen Getränk. Hierzu wird mindestens ein Oxo-Terpen-Carbinol-Derivat verwendet, wobei eines der verschiedenen benutzten Derivate 4,8-Dimethyl-3,7-nonadien-2-ol ist. Jedoch wird der Einsatz eines bestimmten Isomers dieser Verbindung nicht beschrieben.

Eine Modifikation einer funktionellen Gruppe kann auch bei sonst strukturell ähnlichen Verbindungen zu deutlich unterschiedlichen olfaktorischen Eigenschaften führen.

Die olfaktorischen Eigenschaften von 4,8-Dimethyl-3,7-nonadien-2-ol werden als "Narzisse" beschrieben, wohingegen 4,8-Dimethyl-3,7-nonadien-2-on "fruchtig" riecht. Generell werden die Düfte der sekundären Alkohole als unangenehm beschrieben, während die entsprechenden Acetate und Ketone angenehme Düfte besitzen (Indian Perfumer, 1983, 27, 112-18).

Die Veröffentlichung "Synthesis and odor characteristics of some homologs of acyclic terpenoids" von C. Wawrzenczyk in "Parfumer and Flavorist" (1983) beschreibt den Geruch einer Mischung aus 60% (E)-4,8-Dimethyl-3,7-nonadien-2-ol und 40% (Z)-4,8-Dimethyl-3,7-nonadien-2-ol als intensiv, beißend und nach Pilz riechend.

Auf den Seiten 440 und 441 der Veröffentlichung "Sur certaines causes des odeurs gèraniques" von G. Austerweil in "Comptes rendus hebdomadaires de sciences" (1910) wird durch die Umsetzung von Citral mit einem Grignardreagenz 1-Methylgeraniol gewonnen, welches nach "Pelargonie" riecht. Citral ist eine Isomerenmischung von Geranial und Neral. Bei der Umsetzung mit Grignardreagenzien bleibt die E/Z-Isomerenverteilung des Ausgangsmaterials erhalten.

Demnach sind zwar Mischungen der Isomere von 4,8-Dimethyl-3,7-nonadien-2-ol im Stand der Technik beschrieben, allerdings gibt es keine einheitliche Auskunft über den Geruch dieser Mischungen. Eine Mischung aus 60% (E)- und 40% (Z)-Isomeren wurde als unangenehm riechend klassifiziert.

Ein erster Gegenstand der vorliegenden Erfindung ist die Verwendung von 4,8-Dimethyl-3,7-nonadien-2-ol als Riechstoff, worin das Gewichtsverhältnis von (E)-4,8-Dimethyl-3,7-nonadien-2-ol zur Summe von (E)- und (Z)-4,8-Dimethyl-3,7-nonadien-2-ol mindestens 80% beträgt.

Ein weiterer Gegenstand der vorliegenden Erfindung ist die Verwendung von 4,8-Dimethyl-3,7-nonadien-2-ol als Riechstoff, worin das Gewichtsverhältnis von (Z)-4,8-Dimethyl-3,7-nonadien-2-ol zur Summe von (E)- und (Z)-4,8-Dimethyl-3,7-nonadien-2-ol mindestens 80% beträgt.

Die vorliegende Erfindung betrifft außerdem eine Riechstoffkomposition, umfassend 4,8-Dimethyl-3,7-nonadien-2-ol, worin das Gewichtsverhältnis von (E)-4,8-Dimethyl-3,7-nonadien-2-ol zur Summe von (E)- und (Z)-4,8-Dimethyl-3,7-nonadien-2-ol mindestens 80% beträgt.

Die vorliegende Erfindung betrifft außerdem eine Riechstoffkomposition, umfassend 4,8-Dimethyl-3,7-nonadien-2-ol, worin das Gewichtsverhältnis von (Z)-4,8-Dimethyl-3,7-nonadien-2-ol zur Summe von (E)- und (Z)-4,8-Dimethyl-3,7-nonadien-2-ol mindestens 80% beträgt.

Die vorliegende Erfindung betrifft außerdem einen parfümierten Artikel, umfassend 4,8-Dimethyl-3,7-nonadien-2-ol, worin das Gewichtsverhältnis von (E)-4,8-Dimethyl-3,7-nonadien-2-ol zur Summe von (E)- und (Z)-4,8-Dimethyl-3,7-nonadien-2-ol mindestens 80% beträgt.

Die vorliegende Erfindung betrifft außerdem einen parfümierten Artikel, umfassend 4,8-Dimethyl-3,7-nonadien-2-ol, worin das Gewichtsverhältnis von (Z)-4,8-Dimethyl-3,7-nonadien-2-ol zur Summe von (E)- und (Z)-4,8-Dimethyl-3,7-nonadien-2-ol mindestens 80% beträgt.

Die vorliegende Erfindung betrifft auch Verfahren zum Vermitteln, Modifizieren und/oder Verstärken einer rosenartigen Geruchsnote in einer Riechstoffkomposition durch Beimischen einer sensorisch wirksamen Menge von 4,8-Dimethyl-3,7-nonadien-2-ol, worin das Gewichtsverhältnis von (E)-4,8-Dimethyl-3,7-nonadien-2-ol zur Summe von (E)- und (Z)-4,8-Dimethyl-3,7-nonadien-2-ol mindestens 80% beträgt.

Die vorliegende Erfindung betrifft auch Verfahren zum Vermitteln, Modifizieren und/oder Verstärken einer rosenartigen Geruchsnote in einer Riechstoffkomposition durch Beimischen einer sensorisch wirksamen Menge von 4,8-Dimethyl-3,7-nonadien-2-ol, worin das Gewichtsverhältnis von (Z)-4,8-Dimethyl-3,7-nonadien-2-ol zur Summe von (E)- und (Z)-4,8-Dimethyl-3,7-nonadien-2-ol mindestens 80% beträgt.

Die vorliegende Erfindung betrifft auch ein Verfahren zur Herstellung von als Riechstoff geeignetem 4,8-Dimethyl-3,7-nonadien-2-ol durch Umsetzung von 3,7-Dimethylocta-2,6-dienal, worin das Gewichtsverhältnis von (E)-3,7-Dimethylocta-2,6-dienal zur Summe von (E)-3,7-Dimethylocta-2,6-dienal und (Z)-3,7-Dimethylocta-2,6-dienal mindestens 80% beträgt, mit einer methylgruppenübertragenden metallorganischen Verbindung.

Die vorliegende Erfindung betrifft auch ein Verfahren zur Herstellung von als Riechstoff geeignetem 4,8-Dimethyl-3,7-nonadien-2-ol durch Umsetzung von 3,7-Dimethylocta-2,6-dienal, worin das Gewichtsverhältnis von (Z)-3,7-Dimethylocta-2,6-dienal zur Summe von (E)-3,7-Dimethylocta-2,6-dienal und (Z)-3,7-Dimethylocta-2,6-dienal mindestens 80% beträgt, mit einer methylgruppenübertragenden metallorganischen Verbindung.

Als "Riechstoff" sind im Sinne der vorliegenden Erfindung natürliche oder synthetische Stoffe mit Eigengeruch zu verstehen. Der Eigengeruch kann wohlriechend, aversiv oder stinkend sein.

Der "Geruch" oder die "olfaktorische Wahrnehmung" ist im Sinne der vorliegenden Erfindung die Interpretation der Sinnes-Erregungen, die von den Chemorezeptoren der Nase oder anderen Geruchsorganen an das Gehirn eines Lebewesens geliefert werden. Der Geruch kann folglich eine Sinneswahrnehmung der Nase von Riechstoffen sein, die beim Einatmen erfolgt. Die Luft dient hierbei als Geruchsträger.

Als "Duft" ist im Sinne der vorliegenden Erfindung ein wohlriechender Geruch zu verstehen.

(E)-4,8-Dimethyl-3,7-nonadien-2-ol ist eine Verbindung der Formel (I):

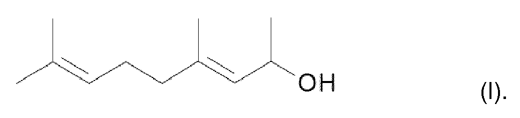

(Z)-4,8-Dimethyl-3,7-nonadien-2-ol ist eine Verbindung der Formel (II):

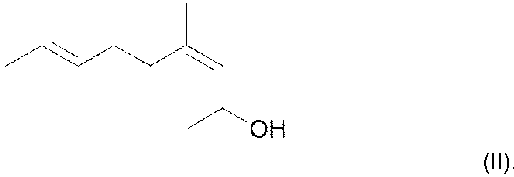

Das erfindungsgemäß zu verwendende 4,8-Dimethyl-3,7-nonadien-2-ol kann als reines Isomer oder als bezüglich eines Isomers angereichertes Isomerengemisch vorliegen. Soweit aus dem Kontext nicht anders ersichtlich, treffen die im Folgenden gemachten Ausführungen und bevorzugten Ausführungsformen für die erfindungsgemäße Verwendung, die erfindungsgemäße Riechstoffkomposition, den erfindungsgemäßen parfümierten Artikel und die erfindungsgemäßen Verfahren zum Vermitteln, Modifizieren und/oder Verstärken einer Geruchsnote zu.

In einer Ausführungsform beträgt das Gewichtsverhältnis von (E)-4,8-Dimethyl-3,7-nonadien-2-ol zur Summe von (E)- und (Z)-4,8-Dimethyl-3,7-nonadien-2-ol mindestens 80%, bevorzugt mindestens 85%, weiterhin bevorzugt mindestens 90%, besonders bevorzugt mindestens 95% weiterhin besonders bevorzugt mindestens 97% und insbesondere bevorzugt 100%. Das Gewichtsverhältnis von (E)-4,8-Dimethyl-3,7-nonadien-2-ol zur Summe von (E)- und (Z)-4,8-Dimethyl-3,7-nonadien-2-ol kann z.B. 80 bis 99% betragen. Ein derartiges Gemisch (einschließlich reinem (E)-4,8-Dimethyl-3,7-nonadien-2-ol) wird im Folgenden als (E)-angereichertes 4,8-Dimethyl-3,7-nonadien-2-ol bezeichnet.

(E)-angereichertes 4,8-Dimethyl-3,7-nonadien-2-ol eignet sich zur Verwendung als Riechstoff, insbesondere zur Erzeugung eines Rosenduftes. Die Tatsache, dass (E)-angereichertes 4,8-Dimethyl-3,7-nonadien-2-ol einen ausdrucksstarken rosenartigen Geruch ohne unerwünschte Noten aufweist ist besonders überraschend, da eine Mischung aus 60% (E)-4,8-Dimethyl-3,7-nonadien-2-ol und 40% (Z)-4,8-Dimethyl-3,7-nonadien-2-ol als nach Pilz riechend klassifiziert wurde.

In einer weiteren Ausführungsform beträgt das Gewichtsverhältnis von (Z)-4,8-Dimethyl-3,7-nonadien-2-ol zur Summe von (E)- und (Z)-4,8-Dimethyl-3,7-nonadien-2-ol mindestens 80%, bevorzugt mindestens 85%, weiterhin bevorzugt mindestens 90%, besonders bevorzugt mindestens 95% weiterhin besonders bevorzugt mindestens 97% und insbesondere bevorzugt 100%. Das Gewichtsverhältnis von (Z)-4,8-Dimethyl-3,7-nonadien-2-ol zur Summe von (E)- und (Z)-4,8-Dimethyl-3,7-nonadien-2-ol kann z.B. 80 bis 99% betragen. Ein derartiges Gemisch (einschließlich reinem (Z)-4,8-Dimethyl-3,7-nonadien-2-ol) wird im Folgenden als (Z)-angereichertes 4,8-Dimethyl-3,7-nonadien-2-ol bezeichnet.

(Z)-angereichertes 4,8-Dimethyl-3,7-nonadien-2-ol eignet sich zur Verwendung als Riechstoff, insbesondere zur Erzeugung eines Rosenduftes. Die Tatsache, dass (Z)-angereichertes 4,8-Dimethyl-3,7-nonadien-2-ol einen ausdrucksstarken rosenartigen Geruch ohne unerwünschte Noten aufweist, ist überraschend, da eine Mischung aus 60% (E)-4,8-Dimethyl-3,7-nonadien-2-ol und 40% (Z)-4,8-Dimethyl-3,7-nonadien-2-ol als nach Pilz riechend klassifiziert wurde.

(E)- oder (Z)-angereichertes 4,8-Dimethyl-3,7-nonadien-2-ol eignen sich für die erfindungsgemäße Verwendung nicht nur aufgrund der beschriebenen Geruchsnoten sondern auch aufgrund ihrer effizienten Herstellungsweise sowie stofflichen Eigenschaften, wie Löslichkeit in gängigen kosmetischen Lösungsmitteln und Kompatibilität mit den gängigen weiteren Bestandteilen derartiger Produkte. Zudem unterstreicht die toxikologische Unbedenklichkeit dieser Riechstoffe ihre besondere Eignung.

Einem weiteren Aspekt zufolge werden die erfindungsgemäß verwendeten Riechstoffe insbesondere zwecks effizienterer Handhabung und Dosierung, auch als Riechstoffmischungen mit Verdünnungs- oder Lösungsmitteln eingesetzt. Hierbei wird der Anteil der Riechstoffe, bezogen auf die Summe von Riechstoffen und Lösungsmittel, in Gew.-% angegeben.

Ein "Lösungsmittel" dient im Sinne der vorliegenden Erfindung der Verdünnung der erfindungsgemäß zu verwendenden Riechstoffe oder der erfindungsgemäßen Riechstoffkompositionen, ohne eigene riechende Eigenschaften zu besitzen. Manche Lösungsmittel haben zugleich fixierende Eigenschaften.

Das (E)- oder (Z)-angereicherte 4,8-Dimethyl-3,7-nonadien-2-ol kann zu 1 bis 99 Gew-% einem Verdünnungs- oder Lösungsmittel beigemischt werden. Bevorzugt sind mindestens 40 Gew.-%ige Lösungen, weiter bevorzugt mindestens 50 Gew.-%ige Lösungen, weiterhin bevorzugt mindestens 60 Gew.-%ige Lösungen, weiter bevorzugt mindestens 70 Gew.-%ige Lösungen, insbesondere bevorzugt mindestens 80 Gew.-%ige Lösungen, weiterhin insbesondere bevorzugt mindestens 90 Gew.-%ige Lösungen, vorzugsweise in parfümistisch akzeptablen Lösungsmitteln.

Bevorzugte parfümistisch akzeptable Lösungsmittel sind Ethanol, Dipropylenglycol (DPG), Propylenglycol, 1,2-Butylenglycol, Glycerin, Diethylenglycolmonoethylether, Diethylphthalat (DEP), Isopropylmyristat (IPM), Triethylcitrat (TEC), Benzylbenzoat (BB) und Benzylacetat. Hierbei wiederum bevorzugt sind Ethanol, Diethylphthalat, Propylenglycol, Dipropylenglycol, Triethylcitrat, Benzylbenzoat und Isopropylmyristat.

Eine "Riechstoffkomposition" ist im Sinne der vorliegenden Erfindung eine Mischung, die neben (E)- oder (Z)-angereichertem 4,8-Dimethyl-3,7-nonadien-2-ol mindestens einen weiteren Riechstoff umfasst. Insbesondere kann es sich bei einer solchen Riechstoffkomposition um eine Parfümkomposition (ein Parfümöl) handeln.

Erfindungsgemäße Riechstoffkompositionen enthalten, bezogen auf die Gesamtmenge der Riechstoffkomposition, z.B. eine Menge von (E)-angereichertem 4,8-Dimethyl-3,7-nonadien-2-ol von 0,01 bis 65 Gew.-%, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%. Das Gewichtsverhältnis von (E)-angereichertem 4,8-Dimethyl-3,7-nonadien-2-ol zu der Gesamtmenge an weiteren (von 4,8-Dimethyl-3,7-nonadien-2-ol verschiedenen) Riechstoffen liegt z.B. im Bereich von 1:1000 bis 1:0,5 liegt, vorzugsweise im Bereich von 1:700 bis 1:1, besonders bevorzugt im Bereich von 1:500 bis 1:10.

Erfindungsgemäße Riechstoffkompositionen enthalten, bezogen auf die Gesamtmenge der Riechstoffkomposition, z.B. eine Menge von (Z)-angereichertem 4,8-Dimethyl-3,7-nonadien-2-ol von 0,01 bis 65 Gew.-% u, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%. Das Gewichtsverhältnis von (Z)-angereichertem 4,8-Dimethyl-3,7-nonadien-2-ol zu der Gesamtmenge an weiteren (von 4,8-Dimethyl-3,7-nonadien-2-ol verschiedenen) Riechstoffen liegt z.B. im Bereich von 1:1000 bis 1:0,5 liegt, vorzugsweise im Bereich von 1:700 bis 1:1, besonders bevorzugt im Bereich von 1:500 bis 1:10.

Ein "Parfüm" ist im Sinne der vorliegenden Erfindung ein Gemisch aus Riechstoffen und Alkohol.

Eine "Parfümkomposition" ist im Sinne der vorliegenden Erfindung ein Parfüm, welches unterschiedlich große Mengen harmonisch aufeinander abgestimmter Einzelkomponenten enthält. Die Eigenschaften der Einzelbestandteile werden genutzt, um in der Kombination ein neues Gesamtbild zu schaffen, wobei die Charakteristika der Ingredienzen in den Hintergrund treten, ohne jedoch unterdrückt zu werden.

Ein "Parfümöl" ist im Sinne der vorliegenden Erfindung ein konzentriertes Gemisch aus Duft- und Riechstoffen, die z.B. in alkoholischen Lösungen zur Parfümierung verschiedener Produkte gebraucht werden.

Erfindungsgemäße Riechstoffkomposition enthalten neben (E)- oder (Z)-angereichertem 4,8-Dimethyl-3,7-nonadien-2-ol zumindest einen weiteren Riechstoff, vorzugsweise 2, 3, 4, 5, 6, 7, 8 oder mehr weitere Riechstoffe, wobei weitere Riechstoffe z.B. ausgewählt sind unter:
Alpha-Hexylzimtaldehyd, 2-Phenoxyethylisobutyrat (Phenirat¹), Dihydromyrcenol (2,6-Dimethyl-7-octen-2-ol), Methyldihydrojasmonat (vorzugsweise mit einem Gehalt an cis-Isomerem von mehr als 60 Gew.-%) (Hedione⁹, Hedione HC⁹), 4,6,6,7,8,8-Hexamethyl-1,3,4,6,7,8-hexahydrocyclopenta[g]benzopyran (Galaxolid³), Tetrahydrolinalool (3,7-Dimethyloctan-3-ol), Ethyllinalool, Benzylsalicylat, 2-Methyl-3-(4-tert-butylphenyl)propanal (Lilial²), Zimtalkohol, 4,7-Methano-3a,4,5,6,7,7a-hexahydro-5-indenylacetat und/oder 4,7-Methano-3a,4,5,6,7,7a hexahydro-6-indenylacetat (Herbaflorat¹), Citronellol, Citronellylacetat, Tetrahydrogeraniol, Vanillin, Linalylacetat, Styrolylacetat (1-Phenylethylacetat), Octahydro-2,3,8,8-tetramethyl-2-acetonaphthon und/oder 2-Acetyl-1,2,3,4,6,7,8-octahydro-2,3,8,8-tetramethyl-naphtalin (Iso E Super³), Hexylsalicylat, 4-tert.-Butylcyclohexylacetat (Oryclone¹), 2-tert.-Butylcyclohexylacetat (Agrumex HC¹), alphalonon (4-(2,2,6-Trimethyl-2-cyclohexen-1-yl)-3-buten-2-on), n-alpha-Methylionon, alpha-Isomethylionon, Coumarin, Terpinylacetat, 2-Phenylethylalkohol, 4-(4-Hydroxy-4-methylpentyl)-3-cyclohexencarboxaldehyd (Lyral³), alpha-Amylzimtaldehyd, Ethylenbrassylat, (E)- und/oder (Z)-3-Methylcyclopentadec-5-enon (Muscenon⁹), 15-Pentadec-11-enolid und/oder 15-Pentadec-12- enolid (Globalide¹), 15-Cyclopentadecanolid (Macrolide¹), 1-(5,6,7,8-Tetrahydro-3,5,5,6,8,8-hexamethyl-2-naphthalenyl) ethanon (Tonalid¹⁰), 2-Isobutyl-4-methyltetrahydro-2H-pyran-4-ol (Florol⁹), 2-Ethyl-4- (2,2,3-trimethyl-3-cyclopenten-1-yl)-2-buten-1-ol (Sandolen¹), cis-3-Hexenylacetat, trans-3-Hexenylacetat, trans-2,cis-6-Nonadienol, 2,4-Dimethyl- 3-cyclohexencarboxaldehyd (Vertocitral¹), 2,4,4,7-Tetramethyl-oct-6-en-3-on (Claritone¹), 2,6-Dimethyl-5-hepten-1-al (Melonal²), Borneol, 3-(3-Isopropylphenyl)butanal (Florhydral²), 2-Methyl-3-(3,4-methylendioxyphenyl)propanal (Helional³), 3-(4-Ethylphenyl)-2,2-dimethylpropanal (Florazon¹), 7-Methyl-2H-1,5-benzodioxepin-3(4H)-on (Calone¹⁹⁵¹⁵), 3,3,5-Trimethylcyclohexylacetat (vorzugsweise mit einem Gehalt an cis-Isomeren von 70 Gew.-%) oder mehr und 2,5,5-Trimethyl-1,2,3,4,4a,5,6,7-octahydronaphthalin-2-ol (Ambrinol S¹). Die vorangehend genannten Riechstoffe werden im Rahmen der vorliegenden Erfindung demnach bevorzugt mit erfindungsgemäßen Mischungen kombiniert.

Sofern vorstehend Handelsnamen angegeben sind, beziehen sich diese auf folgende Quellen:
1 Handelsname Symrise GmbH, Deutschland;
2 Handelsname Givaudan AG, Schweiz;
3 Handelsname International Flavors & Fragrances Inc., USA;
5 Handelsname Danisco Seillans S.A., Frankreich;
9 Handelsname Firmenich S.A., Schweiz;
10 Handelsname PFW Aroma Chemicals B.V., Niederlande.

Weitere Riechstoffe, mit denen (E)- oder (Z)-angereichertes 4,8-Dimethyl-3,7-nonadien-2-ol vorteilhaft zu einer Riechstoffkomposition kombiniert werden können, finden sich z.B. in S. Arctander, Perfume and Flavor Chemicals, Vol. I und II, Montclair, N. J., 1969, Selbstverlag oder K. Bauer, D. Garbe und H. Surburg, Common Fragrance and Flavor Materials, 4rd. Ed., Wiley- VCH, Weinheim 2001. Im Einzelnen seien genannt:
Extrakte aus natürlichen Rohstoffen wie etherischen Ölen, Concretes, Absolues, Resine, Resinoide, Balsame, Tinkturen wie z.B.

Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyl-oxyacetaldehyd; 1-(1-Methoxy-propoxy)-(E/Z)-3-hexen; der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z.B. Geraniol; Nerol; Linalool; Lavadulol; Nerolidol; Farnesol; Tetrahydrolinalool; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z.B. Geranial; Neral; Citronellal; 7-Hydroxy-3,7-dimethyloctanal; 7- Methoxy-3,7-dimethyloctanal; 2,6,1 0-Trimethyl-9-undecenal; Geranylaceton; sowie die Dimethyl- und Diethylacetale von Geranial, Neral, 7-Hydroxy-3,7-dimethyloctanal; der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl- 2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alphan-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1-cyclohexen-1-yl)-2-butenal; Nootkaton; Dihydronootkaton; 4,6,8-Megastigmatrien-3-on; alpha-Sinensal ; beta-Sinensal ; acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3 trimethyl-3-cyclopent-1-yl)-4-penten-2-ol; 1-(2,2,6-Trimethylcyclohexyl)pentan-3-ol; 1-(2,2,6Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclo-dodecan; alpha-Cedrenepoxid; 3a,6,6,9a Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo-[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclo-pentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4 cyclopenta-decenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 7-Cyclohexadecen-1-on; (7/8)-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z.B. 1-(3,3-Dimethylcyclohexyl)-4-penten-1-on; 2,2-Dimethyl-1-(2,4-di methyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclo- pentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7 Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z.B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol, 2-Phenylethylalkohol, 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropa-aldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4 Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; 2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4 indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methyl-N-methylanthranilat; Schiff`'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; 2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2Hfuran-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Ein "Duftthema" ist im Sinne der vorliegenden Erfindung die vorherrschende Duftnote in einer Riechstoffkomposition.

"Fougère" oder eine "Riechstoffkomposition des Fougère-Typs" ein ist im Sinne der vorliegenden Erfindung die Kombination einer frischen Duftnote wie z.B. Citrus, einer floralen, krautigen Duftnote wie z.B. Lavendel, einer orientalischen Duftnote wie z.B. Cumarin mit einer moosigen Duftnote, wie z.B. Eichenmoos. Es können synthetische oder natürliche Riechstoffe eingesetzt werden.

"Frisch" oder eine "Riechstoffkomposition des frischen Typs" ist im Sinne der vorliegenden Erfindung auf die Wirkung der erfindungsgemäß verwendeten Riechstoffe und/oder auf die erfindungsgemäße Riechstoffkomposition bezogen, eine subjektive Empfindung, die durch unterschiedliche Geruchseindrücke bewirkt werden kann. Im europäischen Raum verbindet man mit diesem Begriff vor allem Noten wie Zitrone, Lavendel, helle, blumige Komponenten, also überwiegend leichte, helle Elemente. Es können synthetische oder natürliche Riechstoffe eingesetzt werden.

"Blumig" oder eine "Riechstoffkomposition des blumigen Typs" vereinigt im Sinn der vorliegenden Erfindung den Duft frisch geschnittener Blumen, wie z.B. von Hyazinthe, Lavendel, Maiglöckchen, Rose, Tuberrose, Veilchen, Pelargonie und Jasmin uvm. Blumige Geruchsnoten der vorliegenden Erfindung können auch mit pudrigen Noten wie z.B. Iris und Vanille zu leicht-blumigen Düften oder mit z.B. Orangenblüten, Lindenblüten oder süßen Gewürzen zu blumig-orientalische Düften kombiniert werden. Es können synthetische oder natürliche Riechstoffe eingesetzt werden.

"Blumig-fruchtige Düfte" haben im Sinne der vorliegenden Erfindung einen zusätzlich typprägenden Anteil fruchtiger Noten. Diese bestimmen vor allem die Kopfnoten. Der Schwerpunkt wird dabei immer auf den blumigen Elementen liegen.

Die "Kopfnote" ist im Sinne der vorliegenden Erfindung die erste Phase des Duftablaufs eines Parfüms. Sie spielt die ausschlaggebende Rolle beim ersten Eindruck, beim Öffnen des Flakons und beim Auftragen des Parfüms auf die Haut. Die Aufgabe der Kopfnote ist es, Interesse für das Parfüm allgemein zu wecken und für Aufmerksamkeit zu sorgen. Deshalb ist ein außergewöhnlicher Charakter häufig wichtiger als eine ausgefeilte Harmonie. Die Kopfnote wird naturgemäß von leichtflüchtigen Riechstoffen bestimmt.

Aufgrund der Geruchsnote und stofflichen Eigenschaften eignet sich (E)-angereichertes 4,8-Dimethyl-3,7-nonadien-2-ol besonders gut zur Verwendung als Bestandteil einer Riechstoffkomposition mit rosenartigem, blumigem Duft.

Rosenartige Geruchsnoten werden in vielfältigen Parfümkompositionen und Riechstoffkompositionen eingesetzt, insbesondere z.B. auch in Fougere- oder blumigen (rosigen) Duftthemen. Das überraschenderweise rosige Geruchsprofil von (E)-angereichertem 4,8-Dimethyl-3,7-nonadien-2-ol trägt daher dazu bei, dass dieser Riechstoff für entsprechende Verwendungen in diesen Duftthemen besonders geeignet ist. Vor allem im Zusammenhang mit Riechstoffkompositionen des blumigen (rosigen) oder Fougere-Typs und insbesondere bei der Erzeugung eines Rosenduftes wird eine Vielzahl der positiven Eigenschaften von (E)-angereichertem 4,8-Dimethyl-3,7-nonadien-2-ol besonders deutlich.

(E)-angereichertes 4,8-Dimethyl-3,7-nonadien-2-ol findet vorzugsweise als Bestandteil von Riechstoffkompositionen des Fougere-Typs oder des blumigen (rosigen) Typs Verwendung.

Aufgrund der Geruchsnote und stofflichen Eigenschaften eignet sich (Z)-angereichertes 4,8-Dimethyl-3,7-nonadien-2-ol besonders gut zur Verwendung als Bestandteil einer Riechstoffkomposition mit fruchtig-blumigem oder frischem Duft.

(Z)-angereichertes 4,8-Dimethyl-3,7-nonadien-2-ol findet vorzugsweise als Bestandteil von Riechstoffkompositionen mit blumig-fruchtigen oder frischen Düften Verwendung.

Um eine Riechstoffkomposition mit möglichst geraniolähnlichen Geruchseigenschaften zu erhalten, wird (Z)-angereichertes 4,8-Dimethyl-3,7-nonadien-2-ol oder (E)-angereichertes 4,8-Dimethyl-3,7-nonadien-2-ol mit Tetrahydrogeraniol (3,7-Dimethyloctan-1-ol) kombiniert, wobei bevorzugt (Z)-angereichertes 4,8-Dimethyl-3,7-nonadien-2-ol verwendet wird. Gemäß einer bevorzugten Ausführungsform dieser Riechstoffkomposition wird zusätzlich Citronellol (3,7-Dimethyl-6-octen-1-ol) verwendet. Gemäß einer besonders bevorzugten Ausführungsform dieser Riechstoffkomposition wird neben Citronellol zusätzlich mindestens einen weiteren Riechstoff verwendet, der unter Citronellylacetat (3,7-Dimethyl-6-octen-1-ol-acetat) und 2-Phenylethylalkohol ausgewählt ist.

Entsprechend betrifft ein bevorzugter Aspekt der vorliegenden Erfindung Riechstoffkompositionen, die 3,7-Dimethyloctan-1-ol und (Z)-angereichertes 4,8-Dimethyl-3,7-nonadien-2-ol umfassen. Ein weiterer Aspekt der vorliegenden Erfindung betrifft Riechstoffkompositionen, die 3,7-Dimethyloctan-1-ol und (E)-angereichertes 4,8-Dimethyl-3,7-nonadien-2-ol umfassen. Besonders vorteilhafte Ausführungsformen der vorgenannten Aspekte der Erfindung zum Erzeugen eines geraniolähnlichen Dufts, sind:
Riechstoffkompositionen umfassend 3,7-Dimethyloctan-1-ol, (Z)-angereichertes 4,8-Dimethyl-3,7-nonadien-2-ol und 3,7-Dimethyl-6-octen-1-ol;
Riechstoffkompositionen umfassend 3,7-Dimethyloctan-1-ol, (E)-angereichertes 4,8-Dimethyl-3,7-nonadien-2-ol und 3,7-Dimethyl-6-octen-1-ol;
Riechstoffkompositionen umfassend 3,7-Dimethyloctan-1-ol, (Z)-angereichertes 4,8-Dimethyl-3,7-nonadien-2-ol, 3,7-Dimethyl-6-octen-1-ol und mindestens einen weiteren Riechstoff, der unter 3,7-Dimethyl-6-octen-1-ol-acetat und 2-Phenylethylalkohol ausgewählt ist; sowie
Riechstoffkompositionen umfassend 3,7-Dimethyloctan-1-ol, (E)-angereichertes 4,8-Dimethyl-3,7-nonadien-2-ol, 3,7-Dimethyl-6-octen-1-ol und mindestens einen weiteren Riechstoff, der unter 3,7-Dimethyl-6-octen-1-ol-acetat und 2-Phenylethylalkohol ausgewählt ist.

Bevorzugt umfassen die genannten erfindungsgemäßen Riechstoffkompositionen zum Erzeugen eines geraniolähnlichen Dufts, bezogen auf 100 Gewichtsteile des 4,8-Dimethyl-3,7-nonadien-2-ols:
90-240 Gewichtsteile, insbesondere 120-200 Gewichtsteile und bevorzugt 140-190 Gewichtsteile, z.B. 150-180 Gewichtsteile, des 3,7-Dimethyloctan-1-ols;
sofern vorhanden, 20-100 Gewichtsteile, insbesondere 30-90 Gewichtsteile und bevorzugt 40-80 Gewichtsteile, z.B. 45-70 Gewichtsteile, des 3,7-Dimethyl-6-octen-1-ols;
sofern vorhanden, 7-24 Gewichtsteile, insbesondere 9-20 Gewichtsteile und bevorzugt 11-16 Gewichtsteile, z.B. 12-15 Gewichtsteile, des 3,7-Dimethyl-6-octen-1-ol-acetats; und
sofern vorhanden, 7-24 Gewichtsteile, insbesondere 9-20 Gewichtsteile und bevorzugt 11-16 Gewichtsteile, z.B. 12-15 Gewichtsteile, des 2-Phenylethylalkohols.

In den genannten erfindungsgemäßen Riechstoffkompositionen zum Erzeugen eines geraniolähnlichen Dufts beträgt die Gesamtmenge an 4,8-Dimethyl-3,7-nonadien-2-ol und 3,7-Dimethyloctan-1-ol und, sofern vorhanden, 3,7-Dimethyl-6-octen-1-ol, 3,7-Dimethyl-6-octen-1-ol-acetat und 2-Phenylethylalkohol bevorzugt mindestens 80 Gew.-%, beispielsweise mindestens 90 Gew.-%, mindestens 95 Gew.-%, mindestens 97 Gew.-%, mindestens 98 Gew.-% oder mindestens 99 Gew.-%, und besonders bevorzugt 100 Gew.-%, bezogen auf das Gesamtgewicht der Riechstoffkomposition.

Die oben beschriebenen erfindungsgemäßen Riechstoffkompositionen zum Erzeugen eines geraniolähnlichen Dufts können verwendet werden, um Geraniol oder Geraniolhaltige Zusammensetzungen (z.B. Gemische aus Geraniol und Nerol), insbesondere Zusammensetzungen mit einem Geraniolanteil von mindestens 60 Gew.-%, mindestens 70 Gew.-%, mindestens 80 Gew.-%, mindestens 90 Gew.-%, mindestens 95%, mindestens 96% oder mehr, zu ersetzen. Dabei sind zum Ersatz von Zusammensetzungen mit besonders hohem Geraniolgehalt, wie z.B. von mindestens 80%, mindestens 90%, mindestens 95%, mindestens 96% oder mehr, besonders solche erfindungsgemäßen Riechstoffkompositionen geeignet, die neben (Z)-angereichterem 4,8-Dimethyl-3,7-nonadien-2-ol auch 3,7-Dimethyloctan-1-ol und 3,7-Dimethyl-6-octen-1-ol enthalten.

Die Erfindung betrifft Verfahren zum Vermitteln, Modifizieren und/oder Verstärken einer rosenartigen oder citrusartigen Geruchsnote in einer Riechstoffkomposition.

Die Erfindung betrifft ein Verfahren zum Vermitteln, Modifizieren und/oder Verstärken einer rosenartigen Geruchsnote in einer Riechstoffkomposition durch Beimischen einer sensorisch wirksamen Menge von (E)-angereichertem 4,8-Dimethyl-3,7-nonadien-2-ol.

In Mischung mit anderen Riechstoffen vermag (E)-angereichertes 4,8-Dimethyl-3,7-nonadien-2-ol bereits in geringen Dosierungen der Riechstoffkomposition eine rosenartige Geruchsnote zu verleihen.

(E)-angereichertes 4,8-Dimethyl-3,7-nonadien-2-ol ist für ein Verfahren zum Vermitteln, Modifizieren und/oder Verstärken einer rosenartigen Geruchsnote durch Beimischen einer derartigen Mischung in eine Riechstoffkomposition geeignet.

Demnach wird eine Riechstoffkomposition, die eine organoleptisch/sensorisch wirksame Menge von (E)-angereichertem 4,8-Dimethyl-3,7-nonadien-2-ol enthält, in einem Verfahren verwendet, um eine rosige Geruchsnote zu Vermitteln, Modifizieren und/oder zu Verstärken.

"Modifizieren" bedeutet im Sinne der vorliegenden Erfindung, das Grundthema einer Riechstoffkomposition mit zusätzlichen oder anderen Akkorden und Geruchsnuancen zu versehen.

"Akkorde" ist im Sinne der vorliegenden Erfindung entstehen durch das Zusammenfügen verschiedener Riechstoffe, die sich somit zu neuen Geruchsbildern vereinigen. Die Anzahl der eingesetzten Riechstoffe kann von zwei bis zu mehreren Hunderten reichen.

Eine "organoleptisch/sensorisch wirksame Menge" im Sinne der vorliegenden Erfindung die Menge eines Riechstoffes die ausreicht, um erregend auf ein Sinnesorgan beziehungsweise erregend auf einen sensorischen Rezeptor zu wirken.

Die Erfindung betrifft auch Verfahren zum Vermitteln, Modifizieren und/oder Verstärken einer rosenartigen Geruchsnote in einer Riechstoffkomposition durch Beimischen einer sensorisch wirksamen Menge von (Z)-angereichertem 4,8-Dimethyl-3,7-nonadien-2-ol.

In Mischung mit anderen Riechstoffen vermag (Z)-angereichertes 4,8-Dimethyl-3,7-nonadien-2-ol bereits in geringen Dosierungen der Riechstoffkomposition eine rosenartige und einem weiteren Aspekt zufolge eine frische Geruchsnote zu verleihen.

(Z)-angereichertes 4,8-Dimethyl-3,7-nonadien-2-ol ist für ein Verfahren zum Vermitteln, Modifizieren und/oder Verstärken einer rosenartigen Geruchsnote durch Beimischen einer derartigen Mischung in einer Riechstoffkomposition geeignet.

Demnach wird eine Riechstoffkomposition, die eine organoleptisch/sensorisch wirksame Menge von (Z)-angereichertem 4,8-Dimethyl-3,7-nonadien-2-ol enthält, in einem Verfahren verwendet, um eine rosenartige Geruchsnote zu vermitteln, modifizieren und/oder zu verstärken.

Besonders bevorzugt sind parfümierte Artikel, die eine Menge von (E)-angereichertem 4,8-Dimethyl-3,7-nonadien-2-ol enthalten, die ausreicht, diesem Artikel eine rosenartige Geruchsnote zu vermitteln, zu modifizieren und/oder zu verstärken.

Besonders bevorzugt sind auch parfümierte Artikel, die eine Menge von (Z)-angereichertem 4,8-Dimethyl-3,7-nonadien-2-ol enthalten, die ausreicht, diesem Artikel eine rosenartige Geruchsnote zu vermitteln, zu modifizieren und/oder zu verstärken.

Erfindungsgemäß geeignetes, (E)- oder (Z)-angereichertes 4,8-Dimethyl-3,7-nonadien-2-ol oder erfindungsgemäße Riechstoffkompositionen können in eine Reihe von Produkten eingearbeitet bzw. auf solche Produkte appliziert werden.

Derartige Riechstoffe werden bei der Herstellung parfümierter Artikel eingesetzt. Die olfaktorischen Eigenschaften ebenso wie die stofflichen Eigenschaften (wie Löslichkeit in gängigen Lösungsmitteln und Kompatibilität mit gängigen weiteren Bestandteilen derartiger Produkte) sowie die toxikologische Unbedenklichkeit der erfindungsgemäßen Riechstoffe unterstreichen ihre besondere Eignung für die genannten Einsatzzwecke. Die positiven Eigenschaften tragen dazu bei, dass die erfindungsgemäß verwendeten Riechstoffe und die erfindungsgemäßen Riechstoffkompositionen besonders bevorzugt in Parfümerzeugnissen, Körperpflegeprodukten, Hygieneartikeln, Textilwaschmittel sowie in Reinigungsmitteln für feste Oberflächen eingesetzt werden.

Der parfümierte Artikel ist z.B. ausgewählt unter Parfümerzeugnissen, Körperpflegeprodukten, Hygieneartikeln, Textilwaschmitteln und Reinigungsmitteln für feste Oberflächen. Bevorzugte erfindungsgemäße parfümierte Artikel sind weiterhin ausgewählt unter:
Parfümerzeugnissen, ausgewählt unter Parfüm-Extrakten, Eau de Parfums, Eau de Toilettes, Eau de Colognes, Eau de Solide, Extrait Parfum, Luftverbesserern in flüssiger, gelartiger oder auf einem festen Träger aufgebrachter Form, Aerosolsprays, Duftreinigern und -ölen;
Körperpflegeprodukten, ausgewählt unter Rasierwässern, Pre-shave-Produkten, Splash-Colognes, festen und flüssigen Seifen, Duschgelen, Shampoos, Rasierseifen, Rasierschäumen, Badeölen, kosmetischen Emulsionen vom Öl-in-Wasser-, vom Wasser-in-ÖI- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und - lotionen, Haarpflegeprodukten wie z.B. Haarsprays, Haargelen, festigende Haarlotionen, Haarspülungen, Haarshampoo, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara, Zahnpasta, Zahnseide;
Hygieneartikeln, ausgewählt unter Kerzen, Lampenölen, Räucherstäbchen, Insektiziden, Repellentien, Treibstoffen, Rostentfernern, parfümierten Erfrischungstüchern, Achselpads, Babywindeln, Damenbinden, Toilettenpapier, Kosmetiktüchern, Taschentüchern, Spülmaschinendeo;
Reinigungsmitteln für feste Oberflächen, ausgewählt unter parfümierten sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Wachsen und Polituren wie Möbelpolituren, Fußbodenwachsen, Schuhcremes, Desinfektionsmitteln, Oberflächendesinfektionsmitteln und Sanitärreinigern, Bremsenreinigern, Rohrreinigern, Entkalkern, Grill- und Backofenreinigern, Algen- und Moosentfernern, Schimmelentfernern, Fassadenreinigungsmitteln;
Textilwaschmitteln, ausgewählt unter flüssigen Waschmitteln, pulverförmigen Waschmitteln, Wäschevorbehandlungsmitteln wie Bleichmittel, Einweichmittel und Flecken-entfernern, Wäscheweichspülern, Waschseifen, Waschtabletten.

Einem weiteren Aspekt zufolge sind die erfindungsgemäß verwendeten Riechstoffe und die erfindungsgemäßen Riechstoffkompositionen für den Einsatz in tensidhaltigen parfümierten Artikeln geeignet. Gesucht werden nämlich - insbesondere für die Parfümierung von tensidhaltigen Formulierungen wie zum Beispiel Reinigungsmitteln (insbesondere Geschirrspülmittel und Allzweckreiniger) - häufig Riechstoffe und/oder Riechstoffkompositionen mit einer Rosenkopfnote und ausgeprägter Natürlichkeit.

Einem weiteren Aspekt zufolge können erfindungsgemäß verwendete Riechstoffe und erfindungsgemäße Riechstoffkompositionen als Mittel zum Versehen von (a) Haaren oder (b) textilen Fasern mit der Geruchsnote rosig verwendet werden.

Die erfindungsgemäß zu verwendenden Riechstoffe und erfindungsgemäße Riechstoffkompositionen eignen sich daher besonders gut für den Einsatz in tensidhaltigen parfümierten Artikeln.

Bevorzugt ist es, wenn der parfümierte Artikel einer der folgenden ist:
- ein saures, alkalisches oder neutrales Reinigungsmittel, das insbesondere aus der Gruppe ausgewählt ist bestehend aus Allzweckreinigern, Fußbodenreinigern, Fensterglasreinigern, Geschirrspülmitteln, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Flecken-entfernern, Wäscheweichspülern, Waschseifen, Waschtabletten, Desinfektions-mitteln, Oberflächendesinfektionsmitteln,
- ein Luftverbesserer in flüssiger, gelartiger oder auf einem festen Träger aufgebrachter Form oder als Aerosolspray,
- ein Wachs oder eine Politur, die insbesondere aus der Gruppe ausgewählt ist bestehend aus Möbelpolituren, Fußbodenwachsen und Schuhcremes, oder
- ein Körperpflegemittel, das insbesondere aus der Gruppe ausgewählt ist bestehend aus Duschgelen und Shampoos Rasierseifen, Rasierschäumen, Badeölen, kosmetischen Emulsionen vom Öl-in-Wasser-, vom Wasser-in-ÖI- und vom Wasser-in-ÖI-in-Wasser-Typ wie z.B. Hautcremes- und -lotionen, Gesichtscremes und -lotionen, Sonnenschutzcremes-und -lotionen, After-sun-cremes und -lotionen, Handcremes und - lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik.

Inhaltstoffe, mit denen erfindungsgemäß verwendete Riechstoffe oder erfindungsgemäße Riechstoffkompositionen vorzugsweise kombiniert werden können, sind beispielsweise: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, a-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Einem weiteren Aspekt zufolge verwendet man die Riechstoffe bei der Herstellung der parfümierten Artikel in flüssiger Form, unverdünnt oder mit einem Lösungsmittel verdünnt oder in Form einer Riechstoffkomposition. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw. Für die genannten Lösungsmittel gilt, dass diese, im Falle des Vorhandenseins eigener olfaktorischer Eigenschaften, ausschließlich dem Bestandteil "Lösungsmittel" und nicht den "Riechstoffen" zuzuordnen sind.

Die in den erfindungsgemäßen parfümierten Artikeln enthaltenen Riechstoffe und/oder Riechstoffkompositionen können dabei in einer Ausführungsform an einem Trägerstoff absorbiert sein, der sowohl für eine feine Verteilung des Riechstoffs oder der Riechstoffkomposition im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die erfindungsgemäß verwendeten Riechstoffe und die erfindungsgemäßen Riechstoffkompositionen können auch mikroverkapselt, sprühgetrocknet, als Einschluss-Komplexe oder als Extrusions-Produkte vorliegen und in dieser Form dem zu parfümierenden Produkt, bzw. Artikel, hinzugefügt werden. Die Eigenschaften können durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung kann beispielsweise durch das sogenannte Koazervationsverfahren mit Hilfe von Kapselmaterialien, z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von Riechstoffkompositionen und Cyclodextrinen oder Harnstoffderivaten in ein geeignetes Lösungsmittel, z.B. Wasser, hergestellt werden. Extrusionsprodukte können durch Verschmelzen erfindungsgemäß verwendeter Riechstoffe und erfindungsgemäßer Riechstoffkompositionen mit einem geeigneten wachsartigen Stoff und durch Extrusion mit nachfolgender Erstarrung, ggf. in einem geeigneten Lösungsmittel, z.B. Isopropanol, hergestellt werden.

Erfindungsgemäß geeignetes, (E)- oder (Z)-angereichertes 4,8-Dimethyl-3,7-nonadien-2-ol kann aus einem beliebigen Gemisch von (E)/(Z)-4,8-Dimethyl-3,7-nonadien-2-ol, z.B. dem Umsetzungsprodukt von Citral mit Methylgrignard, durch Isomerentrennung bzw. Isomerenanreicherung erhalten werden. Hierbei kann die Isomerentrennung z.B. durch Rektifikation, Adsorptions- oder Kristallisationsprozesse erreicht werden, vorzugsweise durch Rektifikation.

Die Erfindung betrifft ein Verfahren zur Herstellung von als Riechstoff geeignetem 4,8-Dimethyl-3,7-nonadien-2-ol durch Umsetzung von 3,7-Dimethylocta-2,6-dienal, worin das Gewichtsverhältnis von (E)-3,7-Dimethylocta-2,6-dienal zur Summe von (E)-3,7-Dimethylocta-2,6-dienal und (Z)-3,7-Dimethylocta-2,6-dienal mindestens 80%, bevorzugt mindestens 85%, weiterhin bevorzugt mindestens 90%, besonders bevorzugt mindestens 95% weiterhin besonders bevorzugt mindestens 97% und insbesondere bevorzugt 100%, beträgt, mit einer methylgruppenübertragenden metallorganischen Verbindung.

Die Erfindung betrifft ein Verfahren zur Herstellung von als Riechstoff geeignetem 4,8-Dimethyl-3,7-nonadien-2-ol durch Umsetzung von 3,7-Dimethylocta-2,6-dienal, worin das Gewichtsverhältnis von (Z)-3,7-Dimethylocta-2,6-dienal zur Summe von (E)-3,7-Dimethylocta-2,6-dienal und (Z)-3,7-Dimethylocta-2,6-dienal mindestens 80%, bevorzugt mindestens 85%, weiterhin bevorzugt mindestens 90%, besonders bevorzugt mindestens 95% weiterhin besonders bevorzugt mindestens 97% und insbesondere bevorzugt 100% beträgt, mit einer methylgruppenübertragenden metallorganischen Verbindung.

Die methylgruppenübertragende metallorganische Verbindung ist ausgewählt unter Methyllithium, Cupraten die eine Struktureinheit CH₃-Cu- umfassen, Methylnatrium, Zinkdiorganylen die eine Struktureinheit CH₃-Zn- umfassen, Methylzinkhalogeniden und Methylmagnesiumhalogeniden. Bevorzugt ist die methylgruppenübertragende metallorganische Verbindung ausgewählt unter Methyllithium, Cupraten die eine Struktureinheit CH₃-Cu- umfassen, Zinkdiorganylen die eine Struktureinheit CH₃-Zn- umfassen, und Methylmagnesiumhalogeniden. Methylmagnesiumhalogenide sind besonders bevorzugt, insbesondere Methylmagnesiumbromid und Methylmagnesiumchlorid.

Erfindungsgemäß geeignetes, (E)- oder (Z)-angereichertes 4,8-Dimethyl-3,7-nonadien-2-ol ist durch eine in gängigen Standardwerken der präparative organischen Chemie beschriebene sogenannte "Grignardreaktion" herstellbar.

Die oben beschriebenen Umsetzungen können in einem Temperaturbereich von 10°C bis 70°C erfolgen.

Die Umsetzung erfolgt in der Regel in einem geeigneten inerten Lösungsmittel. Geeignete inerte Lösungsmittel sind z.B. ausgewählt unter Diethylether, Methyl-tert-Butyl Ether, oder Tetrahydrofuran.

Die hydrolytische Aufarbeitung erfolgt in an sich bekannter Weise, z. B. durch Behandlung mit einer verdünnten wässrigen Säure. Dabei wird das Metallalkoholat in den Alkohol umgewandelt.

Nachfolgend wird die Erfindung anhand von Beispielen näher erläutert.

4,8-Dimethyl-3,7-nonadien-2-ole mit unterschiedlichen E/Z-Verhältnissen wurden einem Panel erfahrener Geruchstester vorgelegt. Die Geruchsbeschreibungen sind in der nachstehenden Tabelle zusammengefasst.

### Beispiel 1

| Isomerengemisch | (E)-4,8-Dimethyl-3,7-nonadien-2-ol / (Z)-4,8-Dimethyl-3,7-nonadien-2-ol | Geruchsbeschreibung |
|---|---|---|
| 1_1 | 10/86 | rosig, grün, minzig |
| 1_2 | 15/82 | rosig, floral, grün, minzig |
| 1_3* | 60/39 | rosig, metallisch, krautig, pilzig |
| 1_4 | 85/13 | rosig, floral, citrus, Beinote |
| | | fruchtig (Rhabarber) |
| 1_5 | 98/0 | rosig, floral, citrus, fruchtig (Rhabarber) und krautig |

| | | |
|---|---|---|
| *: nicht erfindungsgemäß | | |

Sämtliche Isomerengemische weisen die begehrte Rosenkomponente auf. Die Gemische 1_1 und 1_2 weisen attraktive frische minzige Beinoten auf. Die Gemische 1_4 und 1_5 weisen attraktive fruchtige Beinoten von Citrus und Rhabarber auf. Das Gemisch 1_3 weist dagegen unerwünschte und unspezifische Noten (pilzig, krautig, flach, metallisch) auf.

Die erfindungsgemäß zu verwendenden Riechstoffe weisen demzufolge einen eindeutigen rosenartigen, floralen Charakter auf. Es treten citrusartige, minzige, grüne, an Rhabarber erinnernde Beinoten auf.

### Beispiel 2

Weiterführende Versuche wurden mit einem Material durchgeführt, welches 86 Gew.-% (Z)-4,8-Dimethyl-3,7-nonadien-2-ol und 10 Gew.-% (E)-4,8-Dimethyl-3,7-nonadien-2-ol enthielt und das im Folgenden als Material A bezeichnet wird.

Zur Bewertung des Geruchs des Materials A wurde dieses in einer repräsentativen Demonstrationsformel geprüft, welche Material A und Verdünnungs-/Lösungsmittel, enthielt. Dabei wurde der Geruch des Materials A wie folgt bewertet: warm, rosig, frisch, mit reicher Blumigkeit, mit leichtem Rosenoxideffekt, mit der typischen fruchtigen Wärme des Geraniols, starke Duftausstrahlung und Duftintensität.

### Beispiel 3

Es wurden Gemische von Material A und verschiedenen weiteren Riechstoffe hergestellt und untersucht. Dabei zeigte sich, dass insbesondere mit den Riechstoffkompositionen der Formeln 2_1 bis 2_5 (siehe nachfolgenden Tabelle) eine starke Annäherung an die Geruchseigenschaften des Geraniols erreicht werden konnte.

| Riechstoff | Formel 2_1 | Formel 2_2 | Formel 2_3 | Formel 2_4 | Formel 2_5 |
|---|---|---|---|---|---|
| 2-Phenylethylalkohol | 4,0 | | | | 4,0 |
| Citronellol | 16,0 | 20,0 | 15,8 | 17,0 | 16,0 |
| Tetrahydrogeraniol | 48,0 | 50,0 | 52,6 | 53,0 | 46,0 |
| Citronellylacetat | 4,0 | | | | 4,0 |
| Material A | 28,0 | 30,0 | 31,6 | 30,0 | 30,0 |

[Mengenangaben in Gew.-% bezogen auf die Gesamtmenge der Riechstoffkomposition.]

Bei den Versuchen mit Material A wurde festgestellt, dass binäre Riechstoffgemische von Tetrahydrogeraniol und Material A deutlich geraniolähnliche Geruchseigenschaften aufweisen. Eine noch stärkere Annäherung an die Geruchseigenschaften des Geraniols wurde mit Riechstoffgemischen erreicht, die Citronellol, Tetrahydrogeraniol und Material A enthielten (wie z.B. den Gemischen der Formeln 2_1 bis 2_5). Unter den untersuchten Riechstoffkompositionen zeigten das Gemisch der Formel 2_5 und insbesondere das der Formel 2_1 die geraniolähnlichsten Geruchseigenschaften.

Demgegenüber fehlte binären Riechstoffgemischen von Citronellol und Tetrahydrogeraniol die typische fruchtige Wärme des Geraniols sowie dessen starke Duftausstrahlung und Duftintensität.

## Patentansprüche

1. Verwendung von 4,8-Dimethyl-3,7-nonadien-2-ol als Riechstoff, worin das Gewichtsverhältnis von (E)-4,8-Dimethyl-3,7-nonadien-2-ol zur Summe von (E)- und (Z)-4,8-Dimethyl-3,7-nonadien-2-ol mindestens 80% beträgt.

2. Verwendung von 4,8-Dimethyl-3,7-nonadien-2-ol als Riechstoff, worin das Gewichtsverhältnis von (Z)-4,8-Dimethyl-3,7-nonadien-2-ol zur Summe von (E)- und (Z)-4,8-Dimethyl-3,7-nonadien-2-ol mindestens 80% beträgt.

3. Verwendung nach Anspruch 1 oder 2 zur Erzeugung eines Rosenduftes.

4. Riechstoffkomposition, umfassend 4,8-Dimethyl-3,7-nonadien-2-ol wie in Anspruch 1 oder 2 definiert.

5. Riechstoffkomposition nach Anspruch 4, ferner umfassend 3,7-Dimethyloctan-1-ol.

6. Riechstoffkomposition nach Anspruch 5, umfassend 90-240 Gewichtsteile 3,7-Dimethyloctan-1-ol bezogen auf 100 Gewichtsteile 4,8-Dimethyl-3,7-nonadien-2-ol.

7. Riechstoffkomposition nach Anspruch 5 oder 6, ferner umfassend 3,7-Dimethyl-6-octen-1-ol.

8. Riechstoffkomposition nach Anspruch 7, umfassend 20-100 Gewichtsteile 3,7-Dimethyl-6-octen-1-ol bezogen auf 100 Gewichtsteile 4,8-Dimethyl-3,7-nonadien-2-ol.

9. Riechstoffkomposition nach einem der Ansprüche 5-8, ferner umfassend mindestens einen weiteren Riechstoff, der unter 3,7-Dimethyl-6-octen-1-ol-acetat und 2-Phenylethylalkohol ausgewählt ist.

10. Riechstoffkomposition nach Anspruch 9, umfassend jeweils 7-24 Gewichtsteile des mindestens einen weiteren Riechstoffs, der unter 3,7-Dimethyl-6-octen-1-ol-acetat und 2-Phenylethylalkohol ausgewählt ist, bezogen auf 100 Gewichtsteile 4,8-Dimethyl-3,7-nonadien-2-ol.

11. Riechstoffkomposition nach einem der Ansprüche 5-10, wobei die Gesamtmenge an 4,8-Dimethyl-3,7-nonadien-2-ol und 3,7-Dimethyloctan-1-ol und, sofern vorhanden, 3,7-Dimethyl-6-octen-1-ol, 3,7-Dimethyl-6-octen-1-ol-acetat und 2-Phenylethylalkohol mindestens 80 Gew.-% der Riechstoffkomposition ausmacht.

12. Riechstoffkomposition nach einem der Ansprüche 5-11, wobei das Gewichtsverhältnis von (Z)-4,8-Dimethyl-3,7-nonadien-2-ol zur Summe von (E)- und (Z)-4,8-Dimethyl-3,7-nonadien-2-ol mindestens 80% beträgt.

13. Parfümierter Artikel, umfassend 4,8-Dimethyl-3,7-nonadien-2-ol wie in Anspruch 1 oder 2 definiert.

14. Parfümierter Artikel nach Anspruch 13, wobei der parfümierte Artikel unter einem Parfümerzeugnis, Körperpflegeprodukt, Hygieneartikel, Textilwaschmittel oder Reinigungsmittel für harte Oberflächen ausgewählt ist.

15. Verfahren zum Vermitteln, Modifizieren und/oder Verstärken einer rosenartigen Geruchsnote in einer Riechstoffkomposition durch Beimischen einer sensorisch wirksamen Menge von 4,8-Dimethyl-3,7-nonadien-2-ol wie in Anspruch 1 oder 2 definiert.

## Claims

1. The use of 4,8-dimethyl-3,7-nonadien-2-ol as fragrance, wherein the weight ratio of (E)-4,8-dimethyl-3,7-nonadien-2-ol to the sum of (E)- and (2)-4,8-dimethyl-3,7-nonadien-2-ol is at least 80%.

2. The use of 4,8-dimethyl-3,7-nonadien-2-ol as fragrance, wherein the weight ratio of (Z)-4,8-dimethyl-3,7-nonadien-2-ol to the sum of (E)- and (2)-4,8-dimethyl-3,7-nonadien-2-ol is at least 80%.

3. The use according to claim 1 or 2 for producing a rose scent.

4. A fragrance composition comprising 4,8-dimethyl-3,7-nonadien-2-ol as defined in claim 1 or 2.

5. The fragrance composition according to claim 4, further comprising 3,7-dimethyl-6-octan-1-ol.

6. The fragrance composition according to claim 5, comprising 90-240 parts by weight of 3,7-dimethyl-6-octan-1-ol, based on 100 parts by weight of 4,8-dimethyl-3,7-nonadien-2-ol.

7. The fragrance composition according to claim 5 or 6, further comprising 3,7-dimethyl-6-octen-1-ol.

8. The fragrance composition according to claim 7, comprising 20-100 parts by weight of 3,7-dimethyl-6-octen-1-ol, based on 100 parts by weight of 4,8-dimethyl-3,7-nonadien-2-ol.

9. The fragrance composition according to any one of claims 5-8, further comprising at least one further fragrance which is selected from 3,7-dimethyl-6-octen-1-ol acetate and 2-phenylethyl alcohol.

10. The fragrance composition according to claim 9, comprising in each case 7-24 parts by weight of the at least one further fragrance which is selected from 3,7-dimethyl-6-octen-1-ol acetate and 2-phenylethyl alcohol, based on 100 parts by weight of 4,8-dimethyl-3,7-nonadien-2-ol.

11. The fragrance composition according to any one of claims 5-10, where the total amount of 4,8-dimethyl-3,7-nonadien-2-ol and 3,7-dimethyl-6-octan-1-ol and, if present, 3,7-dimethyl-6-octen-1-ol, 3,7-dimethyl-6-octen-1-ol acetate and 2-phenylethyl alcohol constitutes at least 80% by weight of the fragrance composition.

12. The fragrance composition according to any one of claims 5-11, where the weight ratio of (Z)-4,8-dimethyl-3,7-nonadien-2-ol to the sum of (E)- and (2)-4,8-dimethyl-3,7-nonadien-2-ol is at least 80%.

13. A perfumed article comprising 4,8-dimethyl-3,7-nonadien-2-ol as defined in claim 1 or 2.

14. The perfumed article according to claim 13, wherein the perfumed article is selected from a perfume product, body care product, hygiene article, textile detergent or cleaner for hard surfaces.

15. A method for conveying, modifying and/or intensifying a rose-like odor note in a fragrance composition by admixing a sensorily effective amount of 4,8-dimethyl-3,7-nonadien-2-ol as defined in claim 1 or 2.

## Revendications

1. Utilisation de 4,8-diméthyl-3,7-nonadién-2-ol en tant que parfum, dans laquelle le rapport pondéral de (E)-4,8-diméthyl-3,7-nonadién-2-ol à la somme de (E)- et (Z)-4,8-diméthyl-3,7-nonadién-2-ol vaut au moins 80 %.

2. Utilisation de 4,8-diméthyl-3,7-nonadién-2-ol en tant que parfum, dans laquelle le rapport pondéral de (Z)-4,8-diméthyl-3,7-nonadién-2-ol à la somme de (E)- et (Z)-4,8-diméthyl-3,7-nonadién-2-ol vaut au moins 80 %.

3. Utilisation selon la revendication 1 ou 2 pour la production d'une odeur de rose.

4. Composition de parfum, comprenant du 4,8-diméthyl-3,7-nonadién-2-ol tel que défini dans la revendication 1 ou 2.

5. Composition de parfum selon la revendication 4, comprenant en outre du 3,7-diméthyloctan-1-ol.

6. Composition de parfum selon la revendication 5, comprenant 90-240 parties en poids de 3,7-diméthyloctan-1-ol par rapport à 100 parties en poids de 4,8-diméthyl-3,7-nonadién-2-ol.

7. Composition de parfum selon la revendication 5 ou 6, comprenant en outre du 3,7-diméthyl-6-octén-1-ol.

8. Composition de parfum selon la revendication 7, comprenant 20-100 parties en poids de 3,7-diméthyl-6-octén-1-ol par rapport à 100 parties en poids de 4,8-diméthyl-3,7-nonadién-2-ol.

9. Composition de parfum selon l'une quelconque des revendications 5 à 8, comprenant au moins un autre parfum qui est choisi parmi l'acétate de 3,7-diméthyl-6-octén-1-ol et l'alcool 2-phényléthylique.

10. Composition de parfum selon la revendication 9, comprenant chaque fois 7-24 parties en poids dudit au moins un autre parfum qui est choisi parmi l'acétate de 3,7-diméthyl-6-octén-1-ol et l'alcool 2-phényléthylique, par rapport à 100 parties en poids de 4,8-diméthyl-3,7-nonadién-2-ol.

11. Composition de parfum selon l'une quelconque des revendications 5 à 10, dans laquelle la quantité totale de 4,8-diméthyl-3,7-nonadién-2-ol et 3,7-diméthyloctan-1-ol et, s'ils sont présents, de 3,7-diméthyloctan-1-ol, acétate de 3,7-diméthyl-6-octén-1-ol et alcool 2-phényléthylique représente au moins 80 % en poids de la composition de parfum.

12. Composition de parfum selon l'une quelconque des revendications 5 à 11, dans laquelle le rapport pondéral de (Z)-4,8-diméthyl-3,7-nonadién-2-ol à la somme de (E)- et (Z)-4,8-diméthyl-3,7-nonadién-2-ol vaut au moins 80 %.

13. Article parfumé, comprenant du 4,8-diméthyl-3,7-nonadién-2-ol tel que défini dans la revendication 1 ou 2.

14. Article parfumé selon la revendication 13, l'article parfumé étant choisi parmi un produit de parfumerie, un produit pour le soin du corps, un article d'hygiène, un produit pour le lavage de textiles ou un produit de nettoyage pour surfaces dures.

15. Procédé pour conférer, modifier et/ou accentuer une note olfactive de type rose dans une composition de parfum, par addition d'une quantité organoleptiquement efficace de 4,8-diméthyl-3,7-nonadién-2-ol tel que défini dans la revendication 1 ou 2.
